(19) Europäisches Patentamt
European Patent Office
Office européen des brevets

(11) **EP 3 748 075 B1**

(12) **EUROPÄISCHE PATENTSCHRIFT**

(45) Veröffentlichungstag und Bekanntmachung des Hinweises auf die Patenterteilung:
**18.08.2021 Patentblatt 2021/33**

(51) Int Cl.:
***D21F 7/00*** *(2006.01)*      ***G01L 5/06*** *(2006.01)*
***G01L 5/103*** *(2020.01)*

(21) Anmeldenummer: **20000203.8**

(22) Anmeldetag: **02.06.2020**

(54) **VERFAHREN ZUM ERFASSEN EINER ZUGSPANNUNG EINES UMLAUFENDEN BANDES**

METHOD FOR MEASURING THE TENSION OF A CIRCULATING BELT

PROCÉDÉ DE MESURE DE TENSION D'UNE BANDE EN CIRCULATION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **07.06.2019 DE 102019004034**

(43) Veröffentlichungstag der Anmeldung:
**09.12.2020 Patentblatt 2020/50**

(73) Patentinhaber: **Texmag GmbH Vertriebsgesellschaft 8800 Thalwil (CH)**

(72) Erfinder: **Wenzkowski, Jürgen 86159 Augsburg (DE)**

(74) Vertreter: **Witzany, Manfred Patentanwalt Falkenstrasse 4 85049 Ingolstadt (DE)**

(56) Entgegenhaltungen:
**DE-A1-102015 008 219     DE-U1- 20 311 822**

**Beschreibung**

Die Erfindung betrifft ein Verfahren zum Erfassen der Zugspannung eines umlaufenden Bandes, welches von mindestens einer Spannwalze umgelenkt wird. Diese Spannwalze ist verstellbar und mit einer Kraftmessvorrichtung verbunden, welche die Lagerkraft der Spannwalze erfasst.

Aus der DE 10 2015 008 219 A1 ist ein gattungsgemäßes Verfahren bekannt. Bei diesem Verfahren wird ein umlaufendes Band von mehreren Walzen umgelenkt, wobei eine davon als Spannwalze verstellbar ist und als Kraftmesswalze ausgebildet ist. Damit ändert sich die Kraftmessrichtung mit der Verstellbewegung, wodurch der von der Kraftmessvorrichtung gemessene Kraftmesswert nicht mehr exakt einer Zugspannung zugeordnet werden kann. Die genannte Druckschrift schlägt daher vor, den Effekt der unterschiedlichen Messrichtung durch eine geeignete Anordnung der Walzen mit einem Effekt aufgrund eines variierenden Umschlingungswinkels der Spannwalze wenigstens teilweise zu kompensieren. Diese Kompensation funktioniert jedoch nicht vollständig, so dass ein systematischer Messfehler verbleibt. Dies wird als nachteilig empfunden. Außerdem gibt es Einbausituationen, in denen die Anordnung gemäß der genannten Druckschrift nicht oder nur mit sehr großem Aufwand realisierbar ist. Auch für derartige Anwendungsfälle möchte man jedoch eine gesicherte Zugspannungserfassung realisieren.

Der Erfindung liegt die Aufgabe zugrunde, ein Verfahren der eingangs genannten Art zu schaffen, das sich durch eine hohe Genauigkeit und breite Anwendbarkeit auszeichnet.

Diese Aufgabe wird erfindungsgemäß mit den folgenden Merkmalen gelöst.

Das erfindungsgemäße Verfahren dient zum Erfassen einer Zugspannung eines umlaufenden Bandes. Dieses umlaufende Band ist vorzugweise als Endlosband realisiert, welches weder Anfang noch Ende besitzt. Derartige Bänder werden hauptsächlich in Papiermaschinen zum Entwässern von Papierbahnen eingesetzt. Das umlaufende Band wird von mindestens einer Spannwalze umgelenkt. Diese mindestens eine Spannwalze ist dabei verstellbar, um auf diese Weise eine Veränderung einer Lauflänge des umlaufenden Bandes zu realisieren. Die konkrete Bahn der Verstellbewegung der Spanwalze ist dabei grundsätzlich beliebig. Wird die mindestens eine Spannwalze tiefer in das umlaufende Band eingetaucht, so erhöht sich zum einen der Umschlingungswinkel der mindestens einen Spannwalze und zum anderen der Weg, also die Lauflänge, die das umlaufende Band entlang eines Umlaufs bewältigen muss. Durch diese Maßnahme kann die Zugspannung des umlaufenden Bandes verändert werden. Um diese Veränderung gezielt vornehmen zu können, ist zusätzlich mindestens eine Kraftmessvorrichtung vorgesehen, die eine Lagerkraft der mindestens einen Spannwalze misst. Die Messung erfolgt dabei in mindestens einer Kraftmessrichtung, die aber wiederum vom Ort der mindestens einen Spannwalze abhängt. Nur auf diese Weise lässt sich ein kompakter Aufbau mit möglichst wenigen Komponenten realisieren, da die mindestens eine Spannwalze sowohl als Aktor zur Veränderung der Zugspannung als auch als Sensor zur Messung derselben dient. Die vielfältigen Änderungen der Umschlingung und Kraftrichtung beeinflussen jedoch das Messergebnis der mindestens einen Kraftmessvorrichtung. Um dennoch eine zuverlässige und reproduzierbare Aussage über die tatsächliche Zugspannung des umlaufenden Bandes zu erzielen, wird die Empfindlichkeit der mindestens einen Kraftmessvorrichtung bezüglich der Zugspannung des umlaufenden Bandes für mehrere Punkte des Verstellweges ermittelt. Diese ermittelten Empfindlichkeiten realisieren gewissermaßen Stützstellen, die den Verlauf der Funktion der Empfindlichkeit vom Verstellweg abbilden. Diese Empfindlichkeiten und/oder daraus berechnete Werte werden als Array in mindestens einem Speicher abgelegt, auf den mindestens ein Controller zugreift. Damit hat der mindestens eine Controller eine Repräsentation der Empfindlichkeitsfunktion vom Verstellweg zur Verfügung, die allerdings nur auf eine diskrete Anzahl von Punkten des Verstellweges beschränkt ist. Die Empfindlichkeit für jeden erdenklichen Punkt des Verstellweges abzulegen, bedarf eines recht großen Speicherbereichs, der in der Regel nicht zur Verfügung steht. Außerdem wäre damit ein erheblicher Rechenaufwand zur Ermittlung der Empfindlichkeiten verbunden. Vielmehr berechnet der mindestens eine Controller aus den gespeicherten Empfindlichkeiten und/oder Werten zusammen mit dem aktuellen Verstellweg der mindestens einen Spannwalze durch Interpolation die Zugspannung. Auf diese Weise ergibt sich ein überraschend geringer Rechenaufwand, so dass verhältnismäßig einfache Controller mit geringer Rechenleistung einsetzbar sind. Zusätzlich bedarf dieses Verfahren nur eines recht überschaubaren Speichers, so dass sich das Verfahren insgesamt effizient und kostengünstig realisieren lässt.

Eine einfache Möglichkeit, die Empfindlichkeiten zu den Punkten des Verstellweges zu ermitteln, ergibt sich, indem sowohl die Kraftmesswerte als auch Zugspannungsmesswerte in Abhängigkeit des Verstellweges ermittelt und daraus die entsprechenden Empfindlichkeiten berechnet werden. Bei dieser experimentellen Vorgangsweise muss für die Ermittlung der Empfindlichkeiten ein zusätzlicher Zugspannungssensor vorgesehen sein, der beispielsweise in das umlaufende Band integriert werden kann. Insbesondere ist hier an einen Dehnungsmessstreifen gedacht. Dieses mit dem zusätzlichen Sensor ausgerüstete Band wird im normalen Betrieb nicht benötigt und dient ausschließlich zur Gewinnung der erforderlichen Empfindlichkeiten. Die Empfindlichkeiten selbst lassen sich einfach als Quotient der Zugspannungsmesswerte zu den Kraftmesswerten berechnen.

Alternativ ist es vorteilhaft, wenn die Empfindlichkeiten zu den Punkten des Verstellweges aus geometrischen Bedingungen berechnet werden. Dabei wird die geometrische Umschlingung der mindestens einen Spannwalze als Funktion des Verstellweges ermittelt. Dies ist zwar mathematisch recht aufwendig, führt jedoch zu exakten Empfindlichkeiten, ohne dass irgendwelche Messfehler bzw. -toleranzen das Ergebnis beeinflussen würden. Der recht hohe, damit verbundene mathematische Aufwand muss auch nur vor Inbetriebnahme durchgeführt werden, da während des Betriebs ausschließlich auf die daraus gewonnenen Empfindlichkeiten Bezug genommen wird.

Für die mathematische Berechnung der Empfindlichkeiten ist es zweckmäßig, die Umschlingungswinkel des Bandes um die mindestens eine Spannwalze und einen Neigungswinkel der mindestens einen Kraftmessvorrichtung zu nutzen. Hieraus lassen sich die gewünschten Empfindlichkeiten berechnen.

Insbesondere, wenn der mindestens eine Controller nur eine geringe Rechenleistung aber relativ viel Speicherplatz zur Verfügung stellt, ist es vorteilhaft, wenn die Interpolation linear erfolgt. Auf diese Weise sind nur wenige und damit schnell ausführbare Rechenoperationen erforderlich. Nachteilig ist jedoch, dass zu einer guten Repräsentation der Empfindlichkeitsfunktion vom Verstellweg eine recht große Anzahl von Empfindlichkeitswerten abgespeichert werden muss.

Eine erhebliche Verbesserung bezüglich des erforderlichen Speicherbedarfs ergibt sich, wenn quadratische Polynome als Interpolationsfunktionen genutzt werden. Dies erhöht zwar den Rechenaufwand, dieser Nachteil wird jedoch durch die wesentlich geringere Anzahl von erforderlichen Empfindlichkeitswerten kompensiert.

In den meisten Anwendungsfällen ist die Funktion der Empfindlichkeiten vom Verstellweg mathematisch gutartig, so dass sie leicht durch ein einziges Polynom interpoliert werden kann. Dieses Polynom hat dann einen Grad, der um 1 kleiner als die Anzahl der gespeicherten Empfindlichkeiten ist. Da sich dieses Polynom dann über den gesamten Verstellbereich erstreckt, ist der Rechenaufwand zur Interpolation überraschend gering. Insbesondere ist zu berücksichtigen, dass nur eine sehr geringe Anzahl von Empfindlichkeiten in diesem Fall notwendig ist, so dass das Interpolationspolynom einen recht überschaubaren Grad aufweist.

Für die Verstellung der mindestens einen Spannwalze hat sich eine Verschwenkung um eine Schwenkachse bewährt. Auf diese Weise ergibt sich eine einfache Lagerung der mindestens einen Spannwalze und gleichzeitig ein kompakter Aufbau. Außerdem kann durch Wahl des Angriffspunktes des Aktors auch eine gewisse Über- bzw. Untersetzung realisiert werden.

Alternativ kann die Verstellung der mindestens einen Spannwalze auch in Form einer Schiebebewegung erfolgen. Hierzu wird vorzugsweise ein Schiebeschlitten eingesetzt, in dem die mindestens eine Spannwalze geführt ist. Dies hat den Vorteil, dass sich der Neigungswinkel der mindestens einen Spannwalze über den Stellweg nicht verändert. Dadurch ergibt sich eine geringere Abhängigkeit der Empfindlichkeit vom Verstellweg. Nachteilig ist jedoch der erhöhte Aufwand zur Realisierung der Lagerung.

Das erfindungsgemäße Verfahren wird beispielhaft anhand der Zeichnung erläutert, ohne den Schutzumfang zu beschränken.

Es zeigt:

Figur 1     eine schematische, räumliche Darstellung einer Vorrichtung zum Spannen eines umlaufenden Bandes in einem ersten Endpunkt,

Figur 2     die Vorrichtung gemäß Figur 1 in einem zweiten Endpunkt,

Figur 3     eine alternative Ausführungsform der Vorrichtung gemäß Figur 1 im ersten Endpunkt,

Figur 4     die Vorrichtung gemäß Figur 3 im zweiten Endpunkt,

Figur 5     eine erste Ausführungsform einer Kraftmessvorrichtung,

Figur 6     eine zweite Ausführungsform einer Kraftmessvorrichtung und

Figur 7     eine Prinzipschaltung eines Controllers.

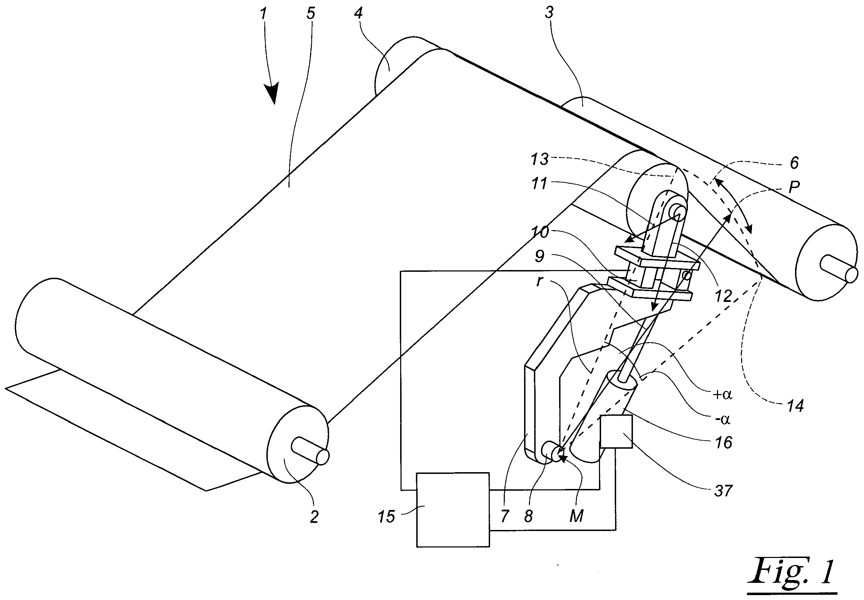

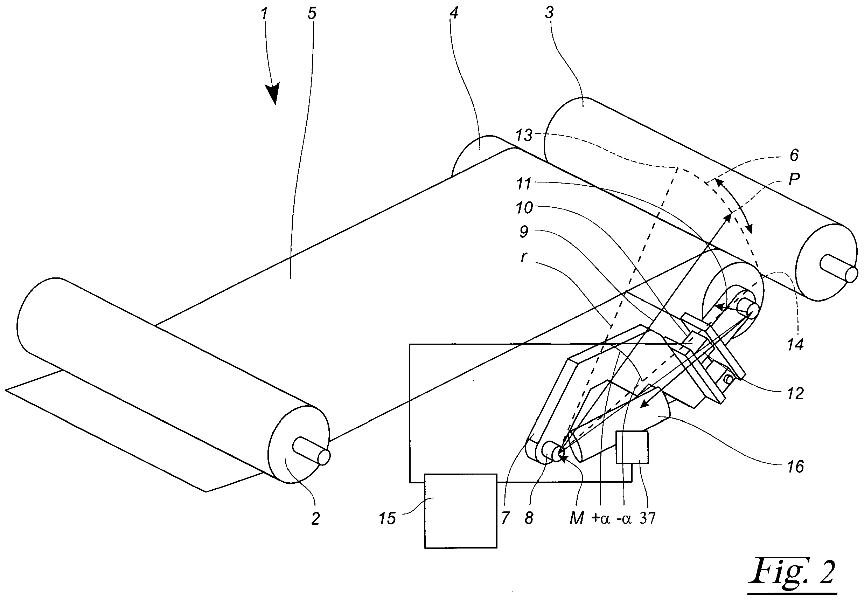

Die Vorrichtung gemäß der Figuren 1 und 2 weist eine erste Leitwalze 2 und eine zweite Leitwalze 3 auf, zwischen denen eine Spannwalze 4 vorgesehen ist. Die Leitwalzen 2, 3 und die Spannwalze 4 lenken ein umlaufendes Band 5 um, wobei die Spannwalze 4 entlang eines Verstellweges 6 verstellbar ist. Hierdurch ist die Länge des Umlaufweges des umlaufenden Bandes 5 einstellbar, um dieses in die nötige Spannung zu versetzen. Zur Verstellung des umlaufenden Bandes 5 ist die Spannwalze 4 auf einem schwenkbaren Stellsupport 7 gehalten. Dieser ist an einer Schwenkachse 8 angelenkt, die einen Mittelpunkt M des Verstellweges 6 bildet. Ein Abstand 9 zwischen der Schwenkachse 8 und einer Außenkontur der Spannwalze 4 bildet einen Radius r des Verstellweges 6.

Diese Schwenklagerung der Spannwalze 4 ist in der Regel sehr einfach zu realisieren, indem an der Schwenkachse 8 ein entsprechendes Drehlager vorgesehen ist. Dies ergibt einen sehr robusten Aufbau, wobei ein Verkanten des Stellsupports 7 beim Auftreten von Kräften, die nicht in Verstellrichtung gerichtet sind, zuverlässig verhindert wird. Ein weiterer Vorteil dieser Geometrie ergibt sich in einem sehr kompakten Aufbau, der insbesondere unter beengten Platzverhältnissen sehr wertvoll ist.

Die Spannwalze 4 ist über eine Kraftmessvorrichtung 10 mit dem Stellsupport 7 gekoppelt. Dieser erfasst die Lagerkraft 12 der Spannwalze 4 in einer Messrichtung 11. Damit erfasst die Kraftmessvorrichtung 10 von der vektoriellen Lagerkraft 12 lediglich jene Komponente, die in Richtung der Messrichtung 11 projiziert ist. Eine senkrecht zur Messrichtung 11 gerichtete Kraftkomponente der Lagerkraft 12 wird dagegen messtechnisch nicht erfasst. In besonderen Einbausituationen kann es durchaus vorkommen, dass die Messrichtung 11 gleich der senkrecht gerichteten Kraftkomponente der Lagerkraft 12 ist. Dies ist jedoch nur in ganz speziellen Einbausituationen der Fall. Im Allgemeinen ist jedoch davon auszugehen, dass eine andere als die gewünschte Kraftkomponente der Lagerkraft 12 gemessen wird.

Die Figur 1 zeigt die Spannwalze 4 in einer ersten Endlage, in der der Stellsupport 7 um +α verschwenkt ist. Die Figur 2 zeigt die Spannwalze 4 in einer zweiten Endlage, in der der Stellsupport 7 um -α verschwenkt ist. Diese Endlagen definieren Endpunkte 13, 14 des Verstellweges 6. Mittig zwischen den Endlagen liegt ein Arbeitspunkt P, der vom Mittelpunkt M der Schwenkbewegung beabstandet ist.

Außerdem ist ein Controller 15 gezeigt, der die Messsignale der Kraftmessvorrichtung 10 und eines Sensors 37 zur Erfassung der Position der Spannwalze 4 erfasst. Der Controller 15 gibt einen Korrekturwert an eine Stellvorrichtung 16 ab, der die Verstellung der Spannwalze 4 bewirkt.

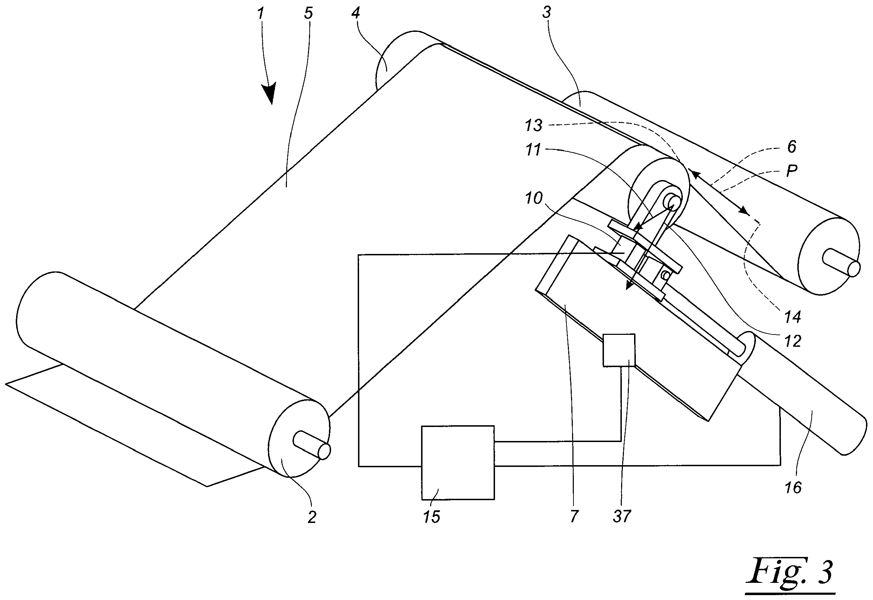

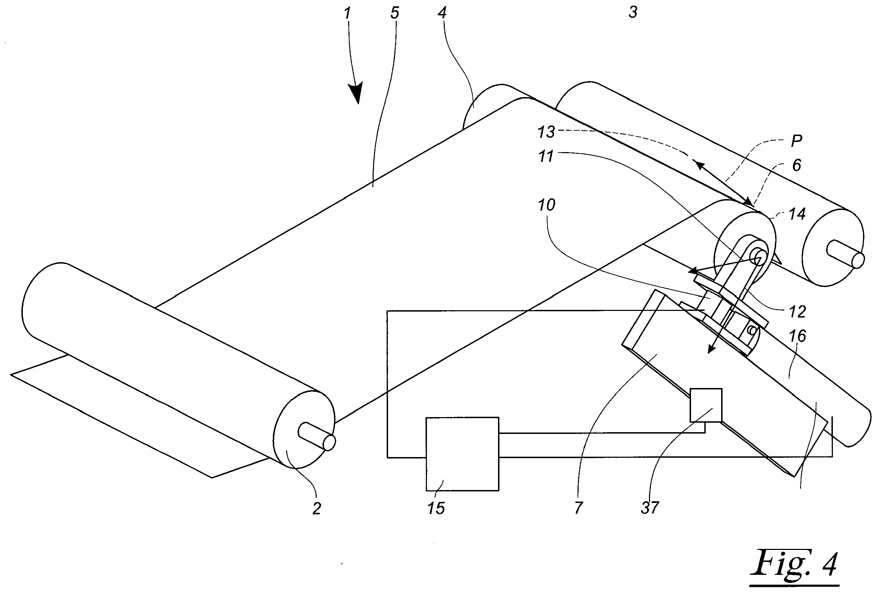

Die Figuren 3 und 4 zeigen eine alternative Ausführungsform der Vorrichtung 1 gemäß den Figuren 1 und 2, wobei gleiche Bezugszeichen gleiche Teile benennen. Im Folgenden wird lediglich auf die Unterschiede zur Ausführungsform gemäß den Figuren 1 und 2 eingegangen.

Bei der Ausführungsform gemäß der Figuren 3 und 4 ist der Stellsupport 7 ein Schiebeschlitten, so dass der Verstellweg 6 eine lineare Verschiebebewegung zwischen den beiden Endpunkten 13, 14 ist. Damit ergeben sich recht einfache geometrische Bedingungen, da die Messrichtung 11 nicht mehr von der Lage der Spannwalze 4 abhängt.

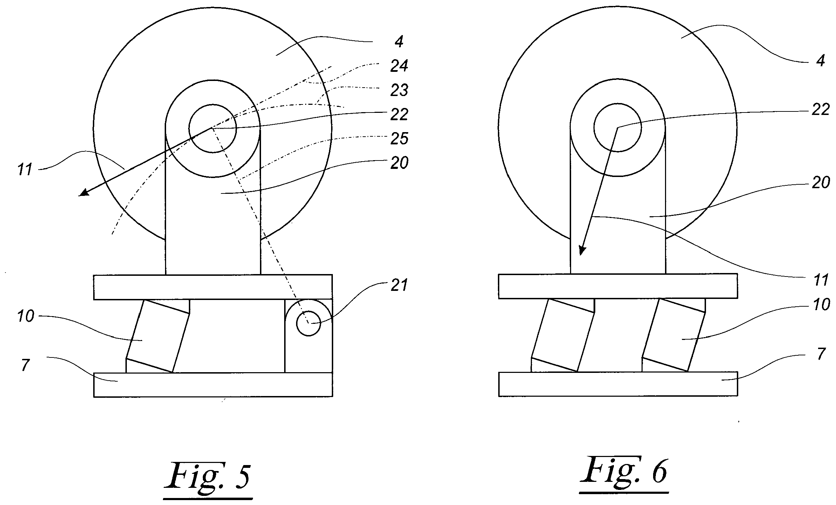

Die Figur 5 zeigt eine erste Ausführungsform einer Spannwalze 4 mit einer Kraftmessvorrichtung 10. Dabei ist die Spannwalze 4 in einem Lagerbock 20 drehbar abgestützt. Der Lagerbock 20 ist über ein Schwenklager 21 mit dem Stellsupport 7 schwenkbar verbunden. Zwischen dem Lagerbock 20 und dem Stellsupport 7 ist die Kraftmessvorrichtung 10 vorgesehen, die die Lagerkraft zwischen dem Lagerbock 20 und dem Stellsupport 7 misst.

Bedingt durch die Schwenklagerung des Lagerbocks 20 lässt sich eine Achse 22 der Spannwalze 4 in Bezug auf die Kraftmessung nur um das Schwenklager 21 verschwenken. Andere Bewegungen der Achse 22 sind nicht möglich. Damit kann die Achse 22 in Bezug auf die Kraftmessung lediglich entlang eines Kreises 23 verstellt werden. Von diesem Kreis 23 wird aber in der Regel nur ein sehr kleiner Ausschnitt tatsächlich realisiert, da übliche Kraftmessvorrichtungen 10 einen verhältnismäßig kleinen Stellweg benötigen. Folglich entspricht der Kreis 23 im Wesentlichen einer Tangente 24, welche den Kreis 23 im Bereich der Achse 22 berührt. Diese Tangente 24 bildet demnach die Messrichtung 11 der Kraftmessvorrichtung 10. Sie ist insbesondere unabhängig von der konkreten Ausrichtung der Kraftmessvorrichtung 10 zwischen dem Lagerbock 20 und dem Stellsupport 7. Demnach könnte die Kraftmessvorrichtung 10 bei dieser Ausführungsform in beliebiger Weise angeordnet sein, ohne die Messrichtung 11 zu beeinflussen. Die Messrichtung 11 wird ausschließlich durch das Schwenklager 21 bestimmt und steht stets senkrecht auf einer Geraden 25 zwischen dem Schwenklager 21 und der Achse 22 der Spannwalze 4. Alternativ können auch mehrere Kraftmessvorrichtungen 10 vorgesehen sein.

Die Figur 6 zeigt eine alternative Ausführungsform der Spannwalze 4 mit der Kraftmessvorrichtung 10, wobei gleiche Bezugszeichen gleiche Teile benennen. Im folgenden wird lediglich auf die Unterschiede zur Ausführungsform gemäß Figur 5 eingegangen.

Bei der Ausführungsform gemäß Figur 6 sind Kraftmessvorrichtungen 10 unmittelbar zwischen dem Lagerbock 20 und dem Stellsupport 7 angeordnet. Damit bestimmen die Kraftmessvorrichtungen 10 selbst die Messrichtung 11.

Im Ausführungsbeispiel gemäß Figur 6 wird angenommen, dass die Kraftmessvorrichtungen 10 lediglich Kräfte in Richtung ihrer Längserstreckung erfassen können, so dass die Messrichtung 11 in diesem Fall leicht zur Senkrechten geneigt ist. Durch anderes Verstellen der Kraftmessvorrichtung 10 kann damit die Messrichtung 11 beliebig verändert werden. Alternativ kann auch nur eine Kraftmessvorrichtung 10 vorgesehen sein.

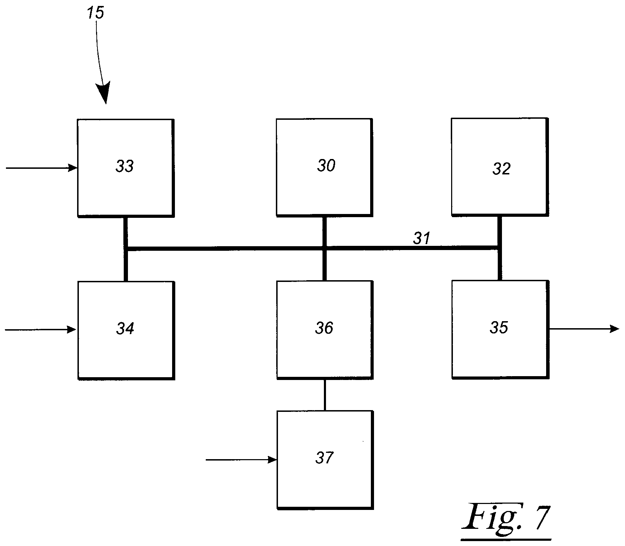

Der Aufbau des Controllers 15 wird anhand der Prinzipschaltung gemäß Figur 7 näher erläutert. Der Controller 15 weist eine CPU 30 auf, die sämtliche Steuer-, Kontroll- und Rechenaufgaben erfüllt. Sie bildet den zentralen Baustein des Controllers 15. Die CPU 30 ist über einen Bus 31 mit einem Speicher 32 verbunden. In diesem Speicher 32 sind ein von der CPU 30 lesbares Programm sowie Daten abgelegt. Über den Bus 31 ist die CPU 30 auch mit Eingabeports 33, 34 sowie einem Ausgabeport 35 verbunden. Der Eingabeport 33 dient zur Eingabe eines Sollwerts für die Zugspannung des umlaufenden Bandes 5. Der Eingabeport 34 nimmt dagegen den Messwert der Kraftmessvorrichtung 10 in analoger oder digitaler Form entgegen. Über den Ausgabeport 35 wird ein Ausgabewert ausgegeben, der direkt die Stellvorrichtung 16 ansteuert.

Der Controller 15 benötigt zur Ermittlung der korrekten Zugspannung noch die Position der Spannwalze 4. Zu diesem Zweck weist der Controller 15 einen weiteren Eingabeport 36 auf, der mit dem Bus 31 verbunden ist. Über diesen Eingabeport 36 erhält der Controller 15 diese Information vom Sensor 37, der unmittelbar die Position der Spannwalze 4 misst. Dieser Sensor 37 ist vorzugsweise ein Winkel- oder Positionsgeber. Für den Fall, dass der Controller 15 zusätzlich auch eine Regelungsfunktion zur Spannkraftregelung übernimmt, kann ggf. auf den Eingabeport 36 und den Sensor 37 verzichtet werden. In diesem Fall ist es durchaus auch vorstellbar, den Ausgabewert zur Ansteuerung der Spannwalze 4 heranzuziehen, um diesen als Positionswert der Spannwalze 4 zu nutzen. Diese Vorgangsweise ist machbar, da die Position der Spannwalze immer in der Nähe des berechneten Korrekturwerts liegen wird. Außerdem spielen geringe Abweichungen in der Position der Spannwalze 4 bei der Berechnung der Zugkraft des umlaufenden Bandes 5 nur eine untergeordnete Rolle. Damit ist ein gewisser Fehler bei der Ermittlung der Position der Spannwalze 4 tolerierbar. Im Falle einer Regelungsfunktion des Controllers 15 können aber trotzdem der Eingabeport 36 und der Sensor 37 realisiert sein. Der damit verbundene Mehraufwand hält sich ohnehin in Grenzen. Außerdem ist hierdurch die gesamte Vorrichtung 1 wesentlich universeller einsetzbar.

Im Speicher 32 sind die Empfindlichkeiten oder Polynomkoeffizienten zur Interpolation zwischen den ermittelten Empfindlichkeiten abgelegt. In letzterem Fall muss die CPU 30 lediglich das Polynom, welches durch die Polynomkoeffizienten repräsentiert wird, für die aktuelle Verstellbewegung auswerten, um auf diese Weise die Empfindlichkeit zu berechnen. Diese kann dann einfach mit dem gemessenen Wert der Kraftmessvorrichtung 10 multipliziert werden. Das Ergebnis dieser Berechnung ist dann die entsprechend korrigierte Zugspannung, die am Ausgabeport 35 ausgegeben wird. Sind dagegen im Speicher 32 die Empfindlichkeiten selbst abgelegt, muss die CPU 30 hieraus die entsprechenden Polynomkoeffizienten in Echtzeit berechnen.

Das Interpolationspolynom berechnet sich gemäß folgender Formel:

$$P_n(\alpha) = \sum_{i=0}^{n} L_i(\alpha)\, e_i$$

Darin sind $e_i$ die bereits vorab ermittelten Empfindlichkeiten und $L_i$ die lagrangeschen Polynome. Die lagrangeschen Polynome berechnen sich gemäß folgender Formel:

$$L_i(\alpha) = \frac{\prod\limits_{j=0, j \neq i}^{n}(\alpha - \alpha_j)}{\prod\limits_{j=0, j \neq i}^{n}(\alpha_i - \alpha_j)}$$

Die Empfindlichkeiten der einzelnen Verstellbewegungen werden entweder experimentell durch Messen der Bandzugspannung oder theoretisch aus der konkreten Geometrie für jede einzelne Verstellbewegung ermittelt. Im letztgenannten Fall wird berechnet, welche Kraft bei jeder einzelnen Verstellbewegung und gegebener Bandzugkraft auf die Kraftmessvorrichtung 10 einwirkt. Die angenommene Bandzugkraft geteilt durch das Ergebnis dieser Berechnung ergibt dann die verstellbewegungsabhängige Empfindlichkeit.

## Bezugszeichenliste

| | | | |
|---|---|---|---|
| 1 | Vorrichtung | M | Mittelpunkt |
| 2 | erste Leitwalze | P | Arbeitspunkt |
| 3 | zweite Leitwalze | r | Radius |
| 4 | Spannwalze | $\alpha$ | Schwenkwinkel |
| 5 | umlaufendes Band | | |
| 6 | Verstellweg | | |
| 7 | Stellsupport | | |
| 8 | Schwenkachse | | |
| 9 | Abstand | | |
| 10 | Kraftmessvorrichtung | | |
| 11 | Messrichtung | | |
| 12 | Lagerkraft | | |
| 13 | erster Endpunkt | | |
| 14 | zweiter Endpunkt | | |
| 15 | Controller | | |
| 16 | Stellvorrichtung | | |
| 20 | Lagerbock | | |
| 21 | Schwenklager | | |
| 22 | Achse | | |
| 23 | Kreis | | |
| 24 | Tangente | | |
| 25 | Gerade | | |
| 30 | CPU | | |
| 31 | Bus | | |
| 32 | Speicher | | |
| 33 | Eingabeport | | |
| 34 | Eingabeport | | |
| 35 | Ausgabeport | | |
| 36 | Eingabeport | | |
| 37 | Sensor | | |

## Patentansprüche

1. Verfahren zum Erfassen einer Zugspannung eines umlaufenden Bandes (5), welches von mindestens einer Spannwalze (4) umgelenkt wird, welche zur Veränderung einer Lauflänge des umlaufenden Bandes (5) um einen Verstellweg (6) verstellt wird, wobei eine Lagerkraft (12) der mindestens einen Spannwalze (4) von mindestens einer Kraftmessvorrichtung (10) in mindestens einer Richtung gemessen wird, welche vom Verstellweg (6) der mindestens einen Spannwalze (4) abhängt, **dadurch gekennzeichnet, dass** für mehrere Punkte des Verstellweges jeweils eine Empfindlichkeit der mindestens einen Kraftmessvorrichtung (10) bezüglich der Zugspannung ermittelt wird, wobei die Empfindlichkeiten und/oder daraus berechnete Werte als Array in mindestens einem Speicher (32) abgelegt werden, auf den mindestens ein Controller (15) zugreift, der aus dem aktuellen Verstellweg (6), der aktuellen Lagerkraft (12) und den gespeicherten Empfindlichkeiten und/oder Werten durch Interpolation die Zugspannung berechnet.

2. Verfahren nach Anspruch 1, **dadurch gekennzeichnet, dass** die Empfindlichkeiten zu den Punkten des Verstellweges aus den Kraftmesswerten und Zugspannungsmesswerten berechnet werden.

3. Verfahren nach Anspruch 1, **dadurch gekennzeichnet, dass** die Empfindlichkeiten zu den Punkten des Verstellweges aus geometrischen Bedingungen berechnet werden.

4. Verfahren nach Anspruch 3, **dadurch gekennzeichnet, dass** die Empfindlichkeiten zu den Punkten des Verstellweges aus Umschlingungswinkeln des Bandes (5) um die mindestens eine Spannwalze (4) und einem Neigungswinkel der mindestens einen Kraftmessvorrichtung (10) berechnet werden.

5. Verfahren nach mindestens einem der Ansprüche 1 bis 4, **dadurch gekennzeichnet, dass** die Interpolation linear erfolgt.

6. Verfahren nach mindestens einem der Ansprüche 1 bis 4, **dadurch gekennzeichnet, dass** die Interpolation quadratisch erfolgt.

7. Verfahren nach mindestens einem der Ansprüche 1 bis 4, **dadurch gekennzeichnet, dass** die Interpolation ein Interpolationspolynom nutzt, welches einen Grad besitzt, der um 1 kleiner als die Anzahl der gespeicherten Empfindlichkeiten ist.

8. Verfahren nach mindestens einem der Ansprüche 1 bis 7, **dadurch gekennzeichnet, dass** die mindestens eine Spannwalze (4) um eine Schwenkachse (8) verschwenkt wird.

9. Verfahren nach mindestens einem der Ansprüche 1 bis 7, **dadurch gekennzeichnet, dass** die mindestens eine Spannwalze (4) linear verschoben wird.

**Claims**

1. Method for measuring the tension of a circulating belt (5) that is deflected by at least one tension roller (4), which is adjusted on an adjustment path (6) to change a running length of the circulating belt (5), a bearing force (12) of the at least one tension roller (4) being measured by at least one force measuring device (10) in at least one direction, which depends on the adjustment path (6) of the at least one tension roller (4), **characterized in that** a respective sensitivity of the at least one force measuring device (10) with respect to the tension is determined for multiple points of the adjustment path, wherein the sensitivities and/or values calculated therefrom are stored as an array in at least one memory (32), to which at least one controller (15) makes access, which calculates the tension from the current adjustment path (6), the current bearing force (12) and the stored sensitivities and/or values by means of interpolation.

2. Method according to Claim 1, **characterized in that** the sensitivities at the points of the adjustment path are calculated from the force measured values and tension measured values.

3. Method according to Claim 1, **characterized in that** the sensitivities at the points of the adjustment path are calculated from geometric conditions.

4. Method according to Claim 3, **characterized in that** the sensitivities at the points of the adjustment path are calculated from wrap angles of the belt (5) about the at least one tension roller (4) and an angle of inclination of the at least one force measuring device (10).

5. Method according to at least one of Claims 1 to 4, **characterized in that** the interpolation is linear.

6. Method according to at least one of Claims 1 to 4, **characterized in that** the interpolation is quadratic.

7. Method according to at least one of Claims 1 to 4, **characterized in that** the interpolation uses an interpolation polynomial which has a degree which is 1 smaller than the number of stored sensitivities.

8. Method according to at least one of Claims 1 to 7, **characterized in that** the at least one tension roller (4) is pivoted about a pivot axis (8).

9. Method according to at least one of Claims 1 to 7, **characterized in that** the at least one tension roller (4) is displaced linearly.

**Revendications**

1. Procédé permettant de détecter une tension d'une bande en circulation (5) qui est renvoyée par au moins un rouleau tendeur (4) qui est réglé selon une course de réglage (6) afin de modifier une longueur de roulement de la bande en circulation (5), une force de support (12) dudit au moins un rouleau tendeur (4) étant mesurée par au moins un dispositif de mesure de force (10) dans au moins un sens qui dépend de la course de réglage (6) dudit au moins un rouleau tendeur (4),

**caractérisé en ce que** pour plusieurs points de la course de réglage, respectivement une sensibilité dudit au moins un dispositif de mesure de force (10) relative à la tension est déterminée,

les sensibilités et/ou les valeurs calculées sur cette base étant enregistrées sous forme de matrice dans au moins une mémoire (32) à laquelle accède au moins un contrôleur (15) qui calcule la tension par interpolation à partir de la course de réglage actuelle (6), de la force de support actuelle (12) et des sensibilités et/ou valeurs mémorisées.

2. Procédé selon la revendication 1, **caractérisé en ce que** les sensibilités concernant les points de la course de réglage sont calculées à partir des valeurs de mesure de force et des valeurs de mesure de tension.

3. Procédé selon la revendication 1, **caractérisé en ce que** les sensibilités concernant les points de la course de réglage sont calculées à partir de conditions géométriques.

4. Procédé selon la revendication 3, **caractérisé en ce que** les sensibilités concernant les points de la course de réglage sont calculées à partir d'angles d'enroulement de la bande (5) autour dudit au moins un rouleau tendeur (4) et à partir d'un angle d'inclinaison dudit au moins un dispositif de mesure de force (10).

5. Procédé selon au moins l'une des revendications 1 à 4, **caractérisé en ce que** l'interpolation est effectuée de manière linéaire.

6. Procédé selon au moins l'une des revendications 1 à 4, **caractérisé en ce que** l'interpolation est effectuée de manière quadratique.

7. Procédé selon au moins l'une des revendications 1 à 4, **caractérisé en ce que** l'interpolation exploite un polynôme d'interpolation qui possède un degré qui est inférieur de 1 au nombre des sensibilités mémorisées.

8. Procédé selon au moins l'une des revendications 1 à 7, **caractérisé en ce que** ledit au moins un rouleau tendeur (4) est amené à pivoter sur un axe de pivotement (8).

9. Procédé selon au moins l'une des revendications 1 à 7, **caractérisé en ce que** ledit au moins un rouleau tendeur (4) est décalé de manière linéaire.

*Fig. 1*

*Fig. 2*

_Fig. 3_

EP 3 748 075 B1

*Fig. 4*

*Fig. 5*

*Fig. 6*

EP 3 748 075 B1

*Fig. 7*

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102015008219 A1